# FASCICULE DE BREVET EUROPEEN

(11) **EP 1 064 945 B1**
(45) Date de publication et mention de la délivrance du brevet: **14.08.2002**
(21) Numéro de dépôt: 00401750.5
(22) Date de dépôt: 20.06.2000
(51) Int. Cl.: A61K 33/00, A61M 16/00, A62B 9/00, B63C 11/00, A62B 7/00, A61P 11/00, A61P 11/06, A61K 47/02

(54) **Utilisations thérapeutiques d'un mélange helium/oxygene, en particulier dans le traitement de l'asthme**
Therapeutische Verwendung eines Helium/Oxygen Gemisches, insbesondere für die Behandlung von Asthma
Therapeutic use of a helium/oxygen mixture, especially for treating asthma

(30) Priorité: 02.07.1999 FR 9908563
(43) Date de publication de la demande: 03.01.2001
(73) Titulaire: AIR LIQUIDE SANTE (INTERNATIONAL), 75007 Paris (FR)
(72) Inventeur: Lecourt, Laurent, 92310 Sevres (FR)
(74) Mandataire: Pittis, Olivier

(56) Documents cités:
- EP-A- 0 244 285
- EP-A- 0 639 378
- EP-A- 0 727 219
- EP-A- 0 868 921
- WO-A-95/16484
- WO-A-95/20411
- WO-A-98/42383
- US-A- 3 682 165
- US-A- 3 815 591
- US-A- 5 228 434
- US-A- 5 542 935
- US-A- 5 570 683
- CHOUX C ET AL: "Helium-oxygen mixture: Pre-hospitalization care of acute asthma: Prospective study: SAMU 69 (October 1991-December 1992)" URGENCES MEDICALES,FR,PARIS, XP002074498 ISSN: 0923-2524
- GLUCK E H ET AL: "HELIUM-OXYGEN MIXTURES IN INTUBATED PATIENTS WITH STATUS ASTHMATICUS AND RESPIRATORY ACIDOSIS" CHEST,US,PARK RIDGE, IL, vol. 98, no. 3, 1 septembre 1990 (1990-09-01), pages 693-698, XP002074496

## Description

L'invention décrit un mélange gazeux binaire constitué d'hélium et d'oxygène et son utilisation pour fabriquer tout ou partie d'un médicament gazeux inhalable destiné à traiter ou prévenir les situations d'insuffisance respiratoire, notamment l'asthme aigu, la broncho-pneumopathie obstructive (BPCO) et toute autre manifestation clinique liée à une obstruction bronchique chez l'homme ou l'animal.

Il est connu que, dans le cas de l'asthme et de plusieurs autres pathologies obstructives analogues, la bronchoconstriction et les remaniements inflammatoires de la muqueuse bronchique, d'une part, et l'hypersécrétion de mucus, d'autre part, engendrent un accroissement des résistances des voies aériennes à l'écoulement des flux gazeux respiratoires, principalement l'air.

Au niveau des voies aériennes d'un être humain ou d'un animal, il est généralement considéré que l'écoulement est laminaire si le nombre de Reynolds est inférieur à 2000 et turbulent si le nombre de Reynolds est supérieur à 4000.

Par exemple, au niveau du bronchospasme, l'écoulement laminaire du gaz devient turbulent.

De ce fait, des résistances aérodynamiques en relation avec cet écoulement sont engendrées et viennent s'ajouter aux résistances aériennes anatomiques.

Cela entraîne alors un surcroît de travail respiratoire de la part du patient car celui-ci est contraint de majorer son travail respiratoire pour réussir à maintenir constants ses débits inspiratoires et expiratoires.

Ce travail excessif va alors entraîner rapidement un surmenage et une fatigue exagérée des muscles respiratoires ; le flux aérien laminaire disparaissant peu à peu.

Une autre conséquence de ce bronchospasme est une moins bonne ventilation du fait du passage moins important d'air et de l'apparition d'une hypoxémie en raison de la quasi constance de l'effet shunt, tel que défini par Physiopathologie Respiratoire ; John B. West ; Ed. Pradel ; Chap. II ; p. 29 ; 1988.

Pour tenter de résoudre ces problèmes, il a déjà été suggéré d'utiliser de l'hélium ou un mélange d'oxygène et d'hélium en tant que gaz respiratoire à administrer au patient pour faciliter l'écoulement gazeux.

En effet, des améliorations cliniques ont pu ainsi être observées avec un mélange hélium/oxygène.

Toutefois, il est à noter que celles-ci son dues exclusivement aux propriétés physiques du mélange hélium/oxygène car l'hélium est un gaz rare, biologiquement inactif qui ne possède aucune activité broncho-dilatatrice ou anti-inflammatoire propre.

La présence d'hélium permet d'abaisser la densité du mélange gazeux.

Il s'ensuit que, dans ces conditions, la valeur du nombre de Reynolds est abaissée du simple fait des caractéristiques de densité du gaz et l'écoulement s'effectue alors selon un régime laminaire en lieu et place d'un régime turbulent.

Le mélange hélium/oxygène améliore le confort du patient, réduit la dyspnée, augmente le temps disponible pour l'expiration et, de ce fait, va augmenter l'élimination du CO₂, ainsi qu'expliqué par le document P. Jolliet ; D. Tassaux ; J.C. Chevrolet ; J. Vincent ; *Ed. Yearbook of intensive care and emergency médecine* ; Berlin ; Heidelberg ; New-York ; London ; Paris : Springer Verlag, 1999 : 244-251.

En outre, on peut citer notamment le document Barach et al. ; *Use of hélium as a new therapeutic gas* ; Proc. Soc. Exp. Bio. Med., 1934 ; 32 : 462-464, qui enseigne un effet bénéfique de mélanges d'oxygène et d'hélium gazeux chez des patients atteints de maladies pulmonaires obstructives.

Par ailleurs, de nombreuses publications traitent de l'utilisation d'hélium gazeux dans l'asthme et les BPCO, mais ces articles sont pour la plupart des séries de cas cliniques, dont la méthodologie d'exécution est souvent critiquable.

Ainsi, on peut citer les documents suivants :
- T.S. Lu, et al. ; *Helium*/*Oxygen in the treatment of upper airway obstruction* ; Anesthesiology 1976; 45 : 678-680 ;
- S.T. Shiue et al. ; *The use of helium-oxygen mixture in the support of patients with status asthmaticus and respiratory acidosis* ; J. Asthma 1989 ; 26: 177-180 ;
- J.E. Kass et al. ; *Heliox therapy in acute sévère asthma* ; Chest 1995 ; 107 : 757-760 ;
- M.R. Wolfson et al. ; *Mechanics and energetics of breathing helium in infants with bronchopulmonary dysplasia* ; J. Pediatr. 1984; 104 : 752-757;
- R.A. Sauder et al. ; *Helium-oxygen and conventional mechanical ventilation in* the *treatment of large airway obstruction and respiratory failure in an infant* ; South. Med. J. ; 1991 ; 84 : 646-648 ;
- C. Elleau et al. ; *Helium-oxygen mixture in respiratory distress syndrome : a double blind study* ; J. Pediatr, 1993 ; 122 : 132-136 ;
- D.M. Swidwa et al. ; Saidel GM ; *Helium-oxygen breathing in severe chronic obstructive pulmonary disease* ; Chest 1985 ; 87 : 790-795 ;
- C.A. Manthous et al. ; *Heliox improves pulsus paradoxus and peak expiratory flow in nonintubated patients with severe asthma* ; Am. J. Respir. Crit. Care Med ; 1995 ; 151 : 310-314 ; et
- F. Martin ; *Utilisation de mélanges Hélium*/*Oxygène au cours de l'état de mal asthmatique* ; Rev. Pneumol. Clin. 1987 ; 43 : 186-189.

En outre, deux publications concernant l'utilisation de mélanges oxygène/hélium chez des patients asthmatiques en crise aiguë, à savoir : R. Carter et al. ; *Evaluation of Heliox in children hospitalized with acute severe* *asthma* ; Chest 1996 ; 109 : 1256-61 et, Kudukis et al. ; *Inhaled Helium-oxygen revisited ; Effect of Inhaled Helium-oxygen during the treatment of status asthmaticus in children* ; J. Pediatr. 1997 ; 130 : 217-24.

L'utilisation d'un mélange avec 80% d'hélium et 20% d'oxygène montre une diminution du pouls paradoxal et une amélioration du débit respiratoire de pointe chez ces patients.

En outre, le document EP-A-741588 décrit l'utilisation d'un gaz renfermant de l'hélium et/ou du néon en tant que vecteur d'aérosol médicamenteux pour le traitement de l'asthme. Selon ce document, la proportion d'hélium dans le gaz est supérieure ou égale à 70%. Il est à noter que des résultats similaires avaient déjà été obtenus et rapportés par le document. M. Svartengren et al. ; *Human Lung Déposition of Particles Suspended in Air or in Helium*/*Oxygen Mixture* ; Exp. Lung. Research, 15 : 575-585, 1989 ; ainsi que par le document A. Malanga et al. ; *Heliox Improves Rate of Response to aérosol bronchodilator* ; Am. Review of Resp. Dis. ; International Conference Supplement, Vol. 147, N° 4, avr. 1993, A65.

Par ailleurs, on peut aussi citer le document EP-A-639378 décrivant l'utilisation thérapeutique d'un mélange gazeux constitué de 30 à 50% en volume d'oxygène et le solde étant de l'hélium additionné de 2 à 20 vpm de monoxyde d'azote (NO).

A l'inverse, le document EP-A-868921 décrit un mélange gazeux comprenant de 20 à 70% d'oxygène, de 1 à 8% de dioxyde de carbone (CO₂) et le complément étant de l'hélium, utilisable pour détecter ou traiter les tumeurs.

Le document EP-A-727219 enseigne, quant à lui, un mélange gazeux constitué de 20 à 40% d'oxygène, de 2 à 10% de dioxyde de carbone et le reste étant de l'hélium, utilisable pour traiter l'asthme.

On peut citer aussi les documents suivants :
- *Helium-Oxygen Mixtures in Intubated Patients with Status Asthmaticus and Respiratiry Acidosis* ; E.H. Gluck et al. ; CHEST / 98 / 3 / Sept. 1990, p. 693-698, décrivant les propriétés physiques de plusieurs mélanges respiratoires constitués de 20% d'hélium et 80% d'oxygène, de 40% d'hélium et 60% d'oxygène, ou de 80% d'hélium et 20% d'oxygène, en particulier l'effet bénéfique de ces mélanges pour réduire les barotraumatismes et améliorer la ventilation des patients.
- US-A-5,228,434 portant sur un gaz anesthésique formé de 60 à 78.5 % molaire de xénon, de 19.5 à 38 % molaire d'oxygène et de 2.5 à 20.5 % molaire d'hélium.
- EP-A-0244285 portant sur l'utilisation de protoxyde d'azote (N₂O) mélangé à de l'oxygène et éventuellement de l'hélium pour réaliser un mélange gazeux non-hypoxiant utilisable en tant que produit de radio sensibilisation des tissus biologiques en radiothérapie.
- *Mélange hélium-oxygène prise en charge préhospitalière des asthmes aigus graves. Etude prospective : SAMU 69*. Conférence BSPP. C. Choux et al., Octobre 1991-Décembre 1992, décrivant l'utilisation de mélanges gazeux binaires constitués de 78% d'hélium et de 22% d'oxygène dans le traitement de l'asthme.
- US-A-3,682,165 portant sur un gaz pour la plongée sous-marine constitué de 60% d'hélium et de 40% d'oxygène.
- US-A-3,815,591 concernant lui aussi un gaz pour la plongée sous-marine constitué de 63% d'hélium et de 37% d'oxygène.

Au vu de l'art antérieur, plusieurs arguments théoriques sont en faveur de l'administration de mélanges gazeux à base d'hélium, à savoir notamment :
. diminution du travail respiratoire
. meilleure épuration du CO₂ expiré au niveau de l'arbre trachéo-bronchique, et
. régime laminaire favorisé.

De plus, en raison de la constance et de l'importance de l'effet shunt, l'addition d'oxygène au mélange gazeux est obligatoire.

Toutefois, comme il résulte des documents ci-dessus, jusqu'à présent, il était usuel et recommandé d'utiliser, pour traiter les patients, des mélanges gazeux contenant au moins 70 à 75% d'hélium et/ou de 20 à 25% d'oxygène, éventuellement additionnés d'autres constituants réputés thérapeutiquement actifs, tels le monoxyde d'azote (NO) comme enseigné par EP-A-639378, ou le dioxyde de carbone (CO₂) comme décrit par EP-A-727219.

Toutefois, il a été mis en évidence que l'utilisation de mélanges classiques à base d'hélium et d'oxygène comportant entre 20 à 25% d'oxygène ne sont pas suffisants pour apporter une oxygénation suffisante du patient dans la majorité des situations.

Il est donc nécessaire d'avoir une deuxième bouteille d'oxygène et d'administrer cet oxygène par une deuxième voie, concomitamment à l'administration du mélange O₂/He.

Cependant, on comprend immédiatement que de devoir rajouter de l'oxygène provenant d'une autre bouteille pose plusieurs problèmes et présente de nombreux inconvénients, notamment :
. difficulté de monitorer correctement la FiO₂, c'est-à-dire la fraction d'oxygène inspirée, réellement administrée au patient,
. risque de perte des bénéfices de l'action de l'hélium : plus le pourcentage en hélium diminue et moins l'effet physique de celui-ci est important,
. risque de toxicité de l'oxygène à forte concentration,
. manipulation, stockage et utilisation de l'oxygène sous surveillance médicale,
. obligation d'avoir deux bouteilles posant des problèmes lors de l'utilisation de ce mélange par des unités mobiles d'intervention d'urgences de type SAMU,
. réglage et calibrage des ventilateurs en ventilation mécanique et non invasive plus difficile,
. sécurité du patient, et
. surveillance accrue par le personnel médical au cours de l'administration au patient.

De là, le but de la présente invention est de résoudre et/ou pallier tout ou partie des inconvénients précités en proposant notamment un mélange gazeux amélioré et son utilisation pour fabrication un médicament gazeux destiné à traiter ou à prévenir les pathologies respiratoires, en particulier l'asthme aigu et les BPCO.

L'invention concerne alors un mélange gazeux binaire prêt à l'emploi constitué de 63.1 à 69.9 % en volume d'hélium et le reste étant de l'oxygène, ledit mélange gazeux étant conditionné à une pression supérieure à 2 bars.

De façon générale, dans le cadre de la présente invention, le mélange gazeux He/O₂ est binaire, c'est-à-dire que la somme des proportions respectives d'hélium et d'oxygène dans le mélange gazeux est approximativement égale et, de préférence, égale à 100% (les % étant des % en volume), à l'exception de la présence possible d'impuretés gazeuses inévitables pouvant résulter du procédé de fabrication du mélange gazeux ou de ses constituants, par exemple des impuretés du type CO, CO₂, H₂O, N₂...

Selon le cas, le mélange gazeux peut comprendre l'une ou plusieurs des caractéristiques suivantes
. il est constitué de 63.2 à 69.8% d'hélium et le solde étant de l'oxygène, de préférence de 63.3 à 69.5% d'hélium, le reste étant de l'oxygène.
. il est constitué de 63.5 à 69% d'hélium et le solde étant de l'oxygène, de préférence il est constitué de 64 à 68% d'hélium, le reste étant de l'oxygène.
. il est constitué de 64 à 67% d'hélium, le reste étant de l'oxygène, de préférence de 64 à 66% d'hélium, le reste étant de l'oxygène.
. il est conditionné à une pression comprise entre 2 et 300 bars, de préférence entre 50 et 300 bars, préférentiellement entre 100 et 250 bars.

L'invention concerne aussi l'utilisation d'un mélange gazeux constitué de 61 à 69.9% en volume d'hélium, de préférence entre 61 et 69% en volume d'hélium, et le reste étant de l'oxygène pour la fabrication d'au moins une partie d'un médicament gazeux inhalable pour traiter ou prévenir une pathologie des voies aériennes supérieures chez l'homme ou l'animal.

Par ailleurs, l'invention a également trait à l'utilisation d'un mélange gazeux constitué de 61 à 69.9% en volume d'hélium, de préférence entre 61 et 69% en volume d'hélium, et le reste étant de l'oxygène pour la fabrication d'au moins une partie d'un médicament gazeux inhalable pour traiter ou prévenir l'asthme, en particulier l'asthme aiguë, chez l'homme ou l'animal.

De manière analogue, l'invention concerne, en outre, l'utilisation d'un mélange gazeux constitué de 61 à 69.9% en volume d'hélium, de préférence entre 61 et 69% en volume d'hélium, et le reste étant de l'oxygène pour la fabrication d'au moins une partie d'un médicament gazeux inhalable pour traiter ou prévenir une broncho-pneumopathie obstructive chez l'homme ou l'animal.

De plus, l'invention a trait à l'utilisation d'un mélange gazeux constitué de 61 à 69.9% en volume d'hélium, de préférence entre 61 et 69% en volume d'hélium, et le reste étant de l'oxygène pour la fabrication d'au moins une partie d'un médicament gazeux pour faciliter ou diminuer le travail respiratoire d'une personnes en phase de sevrage, subséquemment à une période de ventilation mécanique.

En outre, l'invention porte aussi sur l'utilisation d'un mélange gazeux constitué de 61 à 69.9% en volume d'hélium, de préférence entre 61 et 69% en volume d'hélium, et le reste étant de l'oxygène pour la fabrication d'au moins une partie d'un aérosol comportant, en outre, au moins un composé médicamenteux thérapeutiquement efficace, de préférence sous forme liquide et/ou sous forme solide, par exemple une poudre.

Selon un autre aspect, l'invention se rapporte également à un procédé pour convertir un médicament ou un composé médicamenteux en une forme utilisable pour l'administration du médicament par les voies respiratoires des mammifères, dans lequel un liquide et/ou une poudre contenant ou constitué d'au moins un médicament est transformé en un aérosol qui renferme de petites particules de liquide et/ou de poudre à l'aide d'un courant gazeux ou bien à l'aide d'autres moyens, et dans lequel les particules de liquide et/ou de poudre sont recueillies dans le courant gazeux ou bien dans un courant de gaz, respectivement, caractérisé en ce que l'on recueille les particules de liquide dans un mélange gazeux constitué de 61 à 69.9% en volume d'hélium, de préférence entre 61 et 69% en volume d'hélium, et le reste étant de l'oxygène.

Selon encore un autre aspect, l'invention porte sur la préparation médicamenteuse sous forme d'un aérosol comprenant un gaz vecteur et/ou propulseur et des particules liquides et/ou solides renfermant au moins un composé thérapeutiquement efficace, caractérisée en ce que le gaz vecteur et/ou propulseur est un mélange gazeux constitué de 61 à 69.9% en volume d'hélium, de préférence entre 61 et 69% en volume d'hélium, et le reste étant de l'oxygène.

De préférence, le composé thérapeutiquement efficace est choisi parmi les classes thérapeutiques ou médicamenteuses suivantes : β₂ - mimétiques, corticoïdes et anticholinergiques, par exemple, les médicaments commerciaux suivants : BRICANYL™, ADRENALIN™, VENTOLINE™, BECOTIDE™ et LOMUDAL™.

Selon l'utilisation ou la préparation envisagée, le mélange gazeux est constitué de 62 à 68% d'hélium et le solde étant de l'oxygène, de préférence de 63.1 à 68% d'hélium et le solde étant de l'oxygène, de préférence encore de 65% d'hélium et de 35% d'oxygène environ, préférentiellement de 63.5 à 68% d'hélium et le solde étant de l'oxygène, de préférence encore de 64 à 67.5% d'hélium et le solde étant de l'oxygène, de préférence de 65% d'hélium et de 35% d'oxygène environ.

L'invention englobe aussi un récipient de gaz, en particulier une bouteille de gaz, sous pression comprenant un mélange gazeux selon l'invention, ainsi qu'une installation pour administrer un mélange gazeux selon l'invention à un patient, comprenant au moins un récipient de gaz, et au moins un ventilateur d'assistance respiratoire en communication fluidique avec au moins ledit récipient de gaz par l'intermédiaire d'au moins une canalisation de gaz.

De manière analogue, l'invention a également trait à une installation pour administrer un mélange gazeux selon l'invention à un patient, comprenant au moins un récipient de gaz et au moins une interface nasale et/ou buccale, de préférence un masque respiratoire, en communication fluidique avec au moins ledit récipient de gaz par l'intermédiaire d'au moins une canalisation de gaz.

De façon générale, le mélange gazeux 65% He + 35% O₂ de l'invention est préféré.

Des essais cliniques ont permis de montrer l'efficacité des mélanges gazeux selon l'invention lors de leur utilisation dans des situations cliniques liées à une fatigue excessive des muscles respiratoires car l'intérêt du mélange gazeux de l'invention est qu'il permet une moindre fatigue des muscles respiratoires du patient ou du malade, notamment lors de l'utilisation du mélange gazeux de l'invention en phase de post-extubation chez les malades fragilisés par une ventilation mécanique longue ou une ventilation chez des patients en insuffisance respiratoire chronique....

## Revendications

1. Mélange gazeux prêt à l'emploi constitué de 63.1 à 69.9% en volume d'hélium et le reste étant de l'oxygène, ledit mélange gazeux étant conditionné à une pression supérieure à 2 bars.

2. Mélange gazeux selon la revendication 1, **caractérisé en ce qu'**il est constitué de 63.2 à 69.8% en volume d'hélium et le solde étant de l'oxygène, de préférence il est constitué de 63.5 % à 69.5% en volume d'hélium et le solde étant de l'oxygène, de préférence encore il est constitué de 64 à 68% en volume d'hélium, le reste étant de l'oxygène.

3. Mélange gazeux selon l'une des revendications 1 ou 2, **caractérisé en ce qu'**il est constitué de 65% en volume d'hélium et de 35% en volume d'oxygène.

4. Mélange gazeux selon l'une des revendications 1 à 3, **caractérisé en ce qu'**il est conditionné à une pression comprise entre 2 et 300 bars, de préférence à une pression comprise entre 50 et 300 bars.

5. Utilisation d'un mélange gazeux constitué de 61 à 69.9% en volume d'hélium et le solde étant de l'oxygène pour la fabrication d'au moins une partie d'un médicament gazeux inhalable pour traiter ou prévenir une pathologie des voies aériennes supérieures chez l'homme ou l'animal.

6. Utilisation d'un mélange gazeux constitué de 61 à 69.9% en volume d'hélium et le solde étant de l'oxygène pour la fabrication d'au moins une partie d'un médicament gazeux inhalable pour traiter ou prévenir l'asthme chez l'homme ou l'animal, de préférence l'asthme aiguë.

7. Utilisation d'un mélange gazeux constitué de 61 à 69.9% en volume d'hélium et le solde étant de l'oxygène pour la fabrication d'au moins une partie d'un médicament gazeux inhalable pour traiter ou prévenir une broncho-pneumopathie obstructive chez l'homme ou l'animal.

8. Utilisation d'un mélange gazeux constitué de 61 à 69.9% en volume d'hélium et le solde étant de l'oxygène pour la fabrication d'au moins une partie d'un médicament gazeux pour faciliter ou diminuer le travail respiratoire d'une personne en phase de sevrage, subséquemment à une période de ventilation mécanique.

9. Utilisation d'un mélange gazeux constitué de 61 à 69.9% en volume d'hélium et le solde étant de l'oxygène pour la fabrication ou la préparation d'au moins une partie d'un aérosol comportant, en outre, au moins un composé médicamenteux thérapeutiquement efficace.

10. Procédé pour convertir un médicament ou un composé médicamenteux en une forme utilisable pour l'administration du médicament par les voies respiratoires des mammifères, dans lequel un liquide et/ou une poudre contenant ou constitué d'au moins un médicament est transformé en un aérosol qui renferme de petites particules de liquide, et/ou de poudre et dans lequel lesdites particules de liquide et/ou de poudre sont recueillies dans un flux de gaz formé d'un mélange gazeux constitué de 61 à 69.9% en volume d'hélium et le reste étant de l'oxygène.

11. Préparation médicamenteuse sous forme d'un aérosol comprenant un gaz vecteur et/ou propulseur et des particules liquides et/ou solides renfermant au moins un composé thérapeùtiquement efficace, **caractérisée en ce que** le gaz vecteur est un mélange gazeux est constitué de 61 à 69.9% en volume d'hélium et le reste étant de l'oxygène.

12. Préparation selon la revendication 11, **caractérisée en ce que** le composé thérapeutiquement efficace est choisi parmi les β₂ - mimétiques, les corticoïdes et les anticholinergiques.

13. Utilisation selon l'une des revendications 5 à 9, dans laquelle on utilise un mélange gazeux constitué de 62 à 68% en volume d'hélium et le solde étant de l'oxygène, de préférence de 63.1 à 68% en volume d'hélium et le solde étant de l'oxygène.

14. Utilisation selon l'une des revendications 5 à 9, dans laquelle on utilise un mélange gazeux constitué de 63.1 à 68% en volume d'hélium et le solde étant de l'oxygène, de préférence un mélange gazeux constitué de 65% en volume d'hélium et de 35% en volume d'oxygène.

15. Préparation selon l'une des revendications 11 ou 12, **caractérisée en ce que** le mélange gazeux est constitué de 63.5 à 68% en volume d'hélium et le solde étant de l'oxygène, de préférence de 64 à 67.5% en volume d'hélium et le solde étant de l'oxygène.

16. Récipient de gaz, en particulier une bouteille de gaz, sous pression contenant un mélange gazeux d'hélium et d'oxygène selon l'une des revendications 1 à 4.

17. Installation pour administrer un mélange gazeux selon l'une des revendications 1 à 4 à un patient, comprenant au moins un récipient de gaz selon la revendication 16, et au moins un ventilateur d'assistance respiratoire en communication fluidique avec au moins ledit récipient de gaz par l'intermédiaire d'au moins une canalisation de gaz.

18. Installation pour administrer un mélange gazeux selon l'une des revendications 1 à 4 à un patient, comprenant au moins un récipient de gaz selon la revendication 16, et au moins une interface nasale et/ou buccale, de préférence un masque respiratoire, en communication fluidique avec au moins ledit récipient de gaz par l'intermédiaire d'au moins une canalisation de gaz.

19. Préparation médicamenteuse comprenant un mélange gazeux constitué de 61 à 69.9% en volume d'hélium et le reste étant de l'oxygène, et au moins un composé thérapeutiquement efficace choisi parmi les β₂-mimétiques, les corticoïdes et les anti-cholinergiques.

20. Utilisation d'un mélange gazeux constitué d'hélium et d'oxygène, de préférence un mélange gazeux constitué de 61 à 69.9% en volume d'hélium et le reste étant de l'oxygène, et d'au moins un composé thérapeutiquement efficace choisi parmi les β₂-mimétiques, les corticoïdes et les anti-cholinergiques pour fabriquer tout ou partie d'un médicament gazeux inhalable destiné à traiter ou prévenir les situations d'insuffisance respiratoire, notamment l'asthme aigu, la broncho-pneumopathie obstructive (BPCO) et les manifestations cliniques liées à une obstruction bronchique chez l'homme ou l'animal.

## Patentansprüche

1. Gebrauchsfertige gasförmige Mischung aus 63,1 bis 69,9 Vol.-% Helium und Rest Sauerstoff, wobei diese gasförmige Mischung unter einem Druck von über 2 bar verpackt ist.

2. Gasförmige Mischung nach Anspruch 1, **dadurch gekennzeichnet, daß** sie aus 63,2 bis 69,8 Vol.-% Helium und Rest aus Sauerstoff besteht, und zwar vorzugsweise aus 63,5 Vol.-% bis 69,5 Vol.-% aus Helium und Rest Sauerstoff und noch stärker bevorzugt aus 64 bis 68 Vol.-% Helium und Rest Sauerstoff.

3. Gasförmige Mischung nach Anspruch 1 oder 2, **dadurch gekennzeichnet, daß** sie aus 65 Vol.-% Helium und 35 Vol.-% Sauerstoff besteht.

4. Gasförmige Mischung nach einem der Ansprüche 1 bis 3, **dadurch gekennzeichnet, daß** sie unter einem Druck von 2 bis 300 bar, vorzugsweise unter einem Druck von 50 bis 300 bar, verpackt ist.

5. Verwendung einer gasförmigen Mischung aus 61 bis 69,9 Vol.-% Helium und Rest aus Sauerstoff zur Herstellung mindestens eines Teils eines gasförmigen Inhalans-Arzneimittels zur Behandlung oder Vorbeugung einer Pathologie der oberen Atemwege bei Mensch oder Tier.

6. Verwendung einer gasförmigen Mischung aus 61 bis 69,9 Vol.-% Helium und Rest Sauerstoff zur Herstellung mindestens eines Teils eines gasförmigen Inhalans-Arzneimittels zur Behandlung oder Vorbeugung von Asthma, vorzugsweise akutem Asthma, bei Mensch oder Tier.

7. Verwendung einer gasförmigen Mischung aus 61 bis 69,9 Vol.-% Helium und Rest Sauerstoff zur Herstellung mindestens eines Teils eines gasförmigen Inhalans-Arzneimittels zur Behandlung oder Vorbeugung einer obstruktiven Broncho-Pneumopathie bei Mensch oder Tier.

8. Verwendung einer gasförmigen Mischung aus 61 bis 69,9 Vol.-% Helium und Rest Sauerstoff zur Herstellung mindestens eines Teils eines gasförmigen Arzneimittels zur Erleichterung oder Verringerung der Atmungsarbeit eines Menschen im Entwöhnungsstadium im Anschluß an eine Phase mit mechanischer Beatmung.

9. Verwendung einer gasförmigen Mischung aus 61 bis 69,9 Vol.-% Helium und Rest Sauerstoff zur Herstellung mindestens eines Teils eines Aerosols, das weiterhin mindestens eine therapeutisch wirksame Arzneimittelverbindung enthält.

10. Verfahren zum Überführen eines Arzneimittels oder einer Arzneimittelverbindung in eine Form, die zur Verabreichung eines Arzneimittels über die Atemwege von Säugetieren verwendet werden kann, wobei eine Flüssigkeit bzw. ein Pulver, die bzw. das mindestens ein Arzneimittel enthält oder daraus besteht, in ein Aerosol umgewandelt wird, das kleine Flüssigkeits- bzw. Pulverteilchen enthält und in dem diese Flüssigkeits- bzw. Pulverteilchen von einem Gasstrom aufgenommen werden, der aus einer gasförmigen Mischung aus 61 bis 69,9 Vol.-% Helium und Rest Sauerstoff besteht.

11. Arzneimittelpräparat in Form eines Aerosols mit einem Träger- bzw. Treibgas und flüssigen bzw. festen Teilchen, die mindestens eine therapeutisch wirksame Verbindung enthalten, **dadurch gekennzeichnet, daß** es sich bei dem Trägergas um eine gasförmige Mischung handelt, die aus 61 bis 69,9 Vol.-% Helium und Rest Sauerstoff besteht.

12. Präparat nach Anspruch 11, **dadurch gekennzeichnet, daß** die therapeutisch wirksame Verbindung aus den β₂-Mimetika, Corticoiden und Anticholinergika stammt.

13. Verwendung nach einem der Ansprüche 5 bis 9, wobei man eine gasförmige Mischung verwendet, die aus 62 bis 68 Vol.-% Helium und Rest Sauerstoff, vorzugsweise aus 63,1 bis 68 Vol.-% Helium und Rest Sauerstoff, besteht.

14. Verwendung nach einem der Ansprüche 5 bis 9, wobei man eine gasförmige Mischung verwendet, die aus 63,1 bis 68 Vol.-% aus Helium und Rest Sauerstoff besteht, vorzugsweise eine gasförmige Mischung, die aus 65 Vol.-% Helium und 35 Vol.-% Sauerstoff besteht.

15. Präparat nach Anspruch 11 oder 12, **dadurch gekennzeichnet, daß** man eine gasförmige Mischung verwendet, die aus 63,5 bis 68 Vol.-% Helium und Rest Sauerstoff, vorzugsweise aus 64 bis 67,5 Vol.-% Helium und Rest Sauerstoff, besteht.

16. Unter Druck stehendes Gasbehältnis, insbesondere unter Druck stehende Gasflasche, das bzw. die eine gasförmige Mischung aus Helium und Sauerstoff nach einem der Ansprüche 1 bis 4 enthält.

17. Anordnung zur Verabreichung einer gasförmigen Mischung nach einem der Ansprüche 1 bis 4 an einen Patienten mit mindestens einem Gasbehältnis nach Anspruch 16 und mindestens einem Beatmungsgerät zur Unterstützung der Atmung, das mindestens mit dem Gasbehältnis über mindestens eine Gasleitung fluidkommuniziert.

18. Anordnung zur Verabreichung einer gasförmigen Mischung nach einem der Ansprüche 1 bis 4 an einen Patienten mit mindestens einem Gasbehältnis nach Anspruch 16 und mindestens einer Nasen- bzw. Mundschnittstelle, vorzugsweise einer Atemmaske, die mindestens mit dem Gasbehältnis über mindestens eine Gasleitung fluidkommuniziert.

19. Arzneimittelpräparat mit einer gasförmigen Mischung aus 61 bis 69,9 Vol.-% Helium und Rest Sauerstoff und mindestens einer therapeutisch wirksamen Verbindung aus der Gruppe der β₂-Mimetika, Corticoide und Anticholinergika.

20. Verwendung einer gasförmigen Mischung aus Helium und Sauerstoff, vorzugsweise einer gasförmigen Mischung aus 61 bis 69,9 Vol.-% Helium und Rest Sauerstoff, und mindestens einer therapeutisch wirksamen Verbindung aus der Gruppe der β₂-Mimetika, Corticoide und Anticholinergika zur Herstellung eines gesamten gasförmigen Inhalans-Arzneimittels oder eines Teils davon zur Behandlung oder Vorbeugung von Atemnotsituationen, insbesondere akutem Asthma, obstruktiver Broncho-Pneumopathie (COBP) und den mit Bronchialobstruktion assoziierten klinischen Symptomen beim Menschen oder Tier.

## Claims

1. A ready-to-use gaseous mixture consisting of 63.1 to 69.9% by volume of helium and the remainder being oxygen, said gaseous mixture being packaged at a pressure greater than 2 bar.

2. The gaseous mixture as claimed in claim 1, which consists of 63.2 to 69.8% by volume of helium and the balance being oxygen, preferably it consists of 63.5% to 69.5% by volume of helium and the balance being oxygen, still more preferably it consists of 64 to 68% by volume of helium, the remainder being oxygen.

3. The gaseous mixture as claimed in either of claims 1 and 2, which consists of 65% by volume of helium and of 35% by volume of oxygen.

4. The gaseous mixture as claimed in one of claims 1 to 5, which is packaged at a pressure of between 2 and 300 bar, preferably at a pressure of between 50 and 300 bar.

5. Use of a gaseous mixture consisting of 61 to 69.9% by volume of helium and the balance being oxygen, for the manufacture of at least a portion of an inhalable gaseous medicament for treating or preventing a pathology of the upper airways in humans or animals.

6. Use of a gaseous mixture consisting of 61 to 69.9% by volume of helium and the balance being oxygen, for the manufacture of at least a portion of an inhalable gaseous medicament for treating or preventing asthma in humans or animals, preferably acute asthma.

7. Use of a gaseous mixture consisting of 61 to 69.9% by volume of helium and the balance being oxygen, for the manufacture of at least a portion of an inhalable gaseous medicament for treating or preventing an obstructive bronchopneumopathy in humans or animals.

8. Use of a gaseous mixture consisting of 61 to 69.9% by volume of helium and the balance being oxygen, for the manufacture of at least a portion of a gaseous medicament for facilitating or reducing the respiratory work of a person in a weaning phase, subsequent to a period of mechanical ventilation.

9. Use of a gaseous mixture consisting of 61 to 69.9% by volume of helium and the balance being oxygen, for the manufacture or the preparation of at least a portion of an aerosol comprising, in addition, at least one therapeutically effective medicinal compound.

10. A process for converting a medicament or a medicinal compound to a form which can be used for the administration of a medicament by the respiratory tracts of mammals, in which a liquid and/or a powder containing or consisting of at least one medicament is converted to an aerosol which contains small particles of liquid and/or of powder and in which said particles of liquid and/or of powder are collected in a gas stream formed of a gaseous mixture consisting of 61 to 69.9% by volume of helium and the remainder being. oxygen.

11. A medicinal preparation in the form of an aerosol comprising a vector and/or propellant gas and liquid and/or solid particles containing at least one therapeutically effective compound, wherein the vector gas is a gaseous mixture consisting of 61 to 69.9% by volume of helium and the remainder being oxygen.

12. The preparation as claimed in claim 11, wherein the therapeutically effective compound is chosen from β₂-mimetics, corticoids and anticholinergics.

13. The use as claimed in one of claims 5 to 9, in which a gaseous mixture is used which consists of 62 to 68% by volume of helium and the balance being oxygen, preferably from 63.1 to 68% by volume of helium and the balance being oxygen.

14. The use as claimed in one of claims 5 to 9, in which a gaseous mixture is used which consists of 63.1 to 68% by volume of helium and the balance being oxygen, preferably a gaseous mixture which consists of 65% by volume of helium and of 35% by volume of oxygen.

15. The preparation as claimed in either of claims 11 and 12, wherein the gaseous mixture consists of 63.5 to 68% by volume of helium and the balance being oxygen, preferably from 64 to 67.5% by volume of helium and the balance being oxygen.

16. A gas container, in particular a gas bottle, under pressure, containing a gaseous mixture of helium and oxygen as claimed in one of claims 1 to 4.

17. An installation for administering a gaseous mixture as claimed in one of claims 1 to 4 to a patient, comprising at least one gas container as claimed in claim 16, and at least one ventilator for respiratory assistance in fluidic communication with at least said gas container by means of at least one gas pipe.

18. An installation for administering a gaseous mixture as claimed in one of claims 1 to 4 to a patient, comprising at least one gas container as claimed in claim 16, and at least one nasal and/or buccal interface, preferably a respiratory mask, in fluidic communication with at least said gas container by means of at least one gas pipe.

19. A medicinal preparation comprising a gaseous mixture consisting of 61 to 69.9% by volume of helium and the balance being oxygen, and at least one therapeutically effective compound chosen from β₂-mimetics, corticoids and anticholinergics.

20. Use of a gaseous mixture consisting of helium and oxygen, preferably a gaseous mixture consisting of 61 to 69.9% by volume of helium and the remainder being oxygen, and at least one therapeutically effective compound chosen from β₂-mimetics, corticoids and anticholinergics, for the manufacture of the whole or part of an inhalable gaseous medicament intended for treating or preventing respiratory insufficiency situations, in particular acute asthma, obstructive bronchopneumopathy (COBP) and the clinical manifestations linked to a bronchial obstruction in humans or animals.
